Europäisches Patentamt

European Patent Office

Office européen des brevets

(19)

(11) Publication number: **0 161 788**
A1

# (12) EUROPEAN PATENT APPLICATION

(21) Application number: 85302481.8

(22) Date of filing: 09.04.85

(51) Int. Cl.⁴: **C 12 N 15/00**, C 12 N 9/02, C 12 N 1/20, C 12 Q 1/26, C 12 Q 1/68, C 12 P 19/34 // C12R1:19

(30) Priority: 13.04.84 US 600254

(43) Date of publication of application: 21.11.85
Bulletin 85/47

(84) Designated Contracting States: AT BE CH DE FR GB IT LI LU NL SE

(71) Applicant: HOWARD HUGHES MEDICAL INSTITUTE, 3645 Main Highway, Coconut Grove Florida 33133 (US)

(72) Inventor: Woo, Savio L.C., 12806 Palm Desert Lane, Houston Texas 77099 (US)
Inventor: Thirumalachary, T. Chandra c/o T. Venkata Varadan, 24 Palat Sanakran Road Madhavan Colony, Numgampakkam Madras-6000 34 (IN)
Inventor: Lidsky, Alan S., 3755 Merrick, Houston Texas 77025 (US)
Inventor: Robson, Kathryn J.H. Nuffield Department, Clinical Medicine John Radcliffe Hospital, Headington Oxford OX3 (GB)

(74) Representative: Wilkinson, Stephen John et al, c/o Stevens, Hewlett & Perkins 5 Quality Court Chancery Lane, London WC2A 1HZ (GB)

(54) Human phenylalanine hydroxylase cDNA clones, human chromosomal phenylalanine Hydroxylase gene clones and fragments thereof, their methods of production and use in diagnosing classical phenylketonuria.

(57) Described are full-length human phenylalanine hydroxylase cDNA clones, human chromosomal phenylalanine hydroxylase genes and fragments thereof, methods of their production, and the use of these in the diagnosis of the human genetic disorder, classical phenylketonuria (PKU), a hereditary disorder in phenylalanine metabolism that causes permanent mental retardation in humans, and the identification of heterozygous trait carriers as well as other linking genetic disorders. The present invention makes possible mass screening for practically all PKU families.

EP 0 161 788 A1

-1-

IMPROVED HUMAN PHENYLALANINE HYDROXYLASE
cDNA CLONES, HUMAN CHROMOSOMAL PHENYLALANINE
HYDROXYLASE GENE CLONES AND FRAGMENTS THEREOF,
THEIR METHODS OF PRODUCTION AND USE IN
DIAGNOSING CLASSICAL PHENYLKETONURIA

CROSS-REFERENCE TO RELATED APPLICATION

The present application is a continuation-in-part of U.S.A. Application Serial No. 484,816, filed April 14, 1983.

BACKGROUND OF THE INVENTION

Phenylketonuria and its Population Genetics

Phenylketonuria (PKU) is a human genetic disorder caused by an inborn error in aromatic amino acid metabolism. It was first observed some 50 years ago that patients with this condition had excess phenylpyruvate in the urine (Folling, A., 1934a, Nord. Med. Tidskr. 8, 1054-1059; Folling, A., 1934b, Zitschr. Physiol. Chem. 227, 169-176). Subsequently it was discovered that livers of such patients were unable to convert phenylalanine to tyrosine which is the major metabolic pathway of phenylalanine (Jervis, G.A., 1953, Proc. Soc. Exp. Biol. Med. 82, 514-515; Udenfriend, S. and Bessman, S.P., 1953, J. Biol. Chem. 203, 961-966; Wallace et al, 1957, Proc. Soc. Exp. Biol. Med. 94, 632-633; Mitoma et al, 1957, Proc. Sco. Exp. Biol. Med. 94, 634-635). Classical PKU is thus characterized by a lack of phenylalanine hydroxylase activity in the liver (Kaufman, S. 1976, In: Advances in Neurochemistry, Vol. 2, pp. 1-132, Agranoff, B.W. and Aprison, M.H., eds., Plenum Press, New York and London). The lack of this enzymatic activity causes persistent hyperphenylalaninemia and minor metabolic pathways for phenylalanine become over-utilized. High levels of phenylalanine and its derivatives are toxic and cause disturbances in tyrosine and tryptophan metabolism (Blau, K., 1979, In: Aromatic Amino Acid Hydroxylase and Mental Disease [Youdim, M.B.H., ed.] pp. 79-139, New York, Wiley). Diminished formation of catecholamines, melanin and serotonin is typical in untreated PKU patients, and the clinical symptom is an irreversible impairment of brain development resulting in severe mental retardation of the untreated children (Folling, A., 1934a, Nord. Med. Tidskr. 8, 1054-1959; Folling, A., 1934b, Zitschr. Physiol. Chem. 227, 169-176).

Phenylalanine hydroxylase deficiency, regardless of phenotypic variations, is transmitted as an autosomal recessive trait. Massive screening of over 5 million neonates has shown that the prevalence of classical phenylketonuria among Caucasians ranges from 1/5,400 in Ireland to 1/16,600 in Switzerland (Thalhammer et al, 1975, Humangenetik 30, 273-286). The collective frequency in 8 Western European countries and the United States is about 1/8,000 and that 2% of the population are heterozygous carriers of the PKU trait. (Bickel et al, 1973, Acta Paediat. Scand. 62, 413-416; Scriver, C.R. and Rosenberg, L.E., 1973, In: Amino Acid Metabolism and Its Disorders, pp. 290-337, Philadelphia:Saunders, 491 pp.).

Current Methods of Neonatal Screening and Treatment

Phenylketonuric patients secrete large quantities of phenylpyruvate in the urine, which can be readily detected by its reaction with ferric chloride and was used in the 1950's as a screening test for diagnosis of PKU children. A simple and mass screening method for a semi-quantitative determination of phenylalanine in small samples of blood from new-born infants was subsequently developed by Guthrie, R. and Susi, A., 1963, Pediatrics 32, 338-343. PKU neonates identified by this test can be treated with a special protein lysate low in phenylalanine content (Bickel et al, 1954, Acta Paediat. Scand. 43, 64-77; Armstrong, M.D. and Tyler, F.H., 1955, J. Clin. Invest. 34, 565-580). This treatment causes a reduced plasma phenylalanine level, disappearance of phenylpyruvate in urine, and is associated with an improvement in mental development and behavioral performance (Koch et al, 1971, In: Phenylketonuria and Some Other Inborn Errors of Metabolism [Bickel, H., Hudson, F.P. and Woolf, L.I., eds.] pp. 20-25; Wamberg, 1977, In: Medico-Social Management of Inherited Metabolic Disease [Raine, D.N., ed.], pp. 63-78, MTP Press Ltd., Lancaster, England; Smith, I. and Wolff, O.H., 1974, Lancet 2, 540-544).

Early diagnosis of classical PKU and treatment, however, do not achieve uniformly normal IQ scores and learning ability. Initiation of dietary therapy soon after birth has tremendous psychological and social implications for the patient's family (Mikkelson et al, 1974, Naringsforskning 18, 78-86). The dietary correction must be implemented over a prolonged period of time to be effective, and termination of treatment in the mid-first decade is apparently followed by a small but significant decline in IQ scores (Scriver et al, 1980a, N. Eng. J. Med. 303, 1336; Scriver, et al, 1980b, N. Eng. J. Med. 303, 1394). Supervision of this treatment has been shown to be very difficult and can best be performed only at centers experienced with such regimens.

Prior to our earlier application, Serial No. 484,816, there were no prenatal diagnostic methodologies for identification of receissive homozygotes, and the tests available for identification of heterozygotes are not very quantitative (Woolf et al, 1967, Nature 213, 882-885; Rampini, S., 1973, Schweiz Med. Wschr. 103, 537-546; Guttler, F. and Wamberg, E., 1977, Acta Paeeiat, Scand. 66, 339-344; Guttler, F. and Hansen, G., 1977a, Ann.

Clin. Biochem. 14, 124-134; Guttler, F. and Hansen, G., 1977b, Clin. Gen. 11, 137-146). Thus, the development of methodologies for prenatal diagnosis and adult heterozygote detection are desperately needed for prevention, rather than therapy, of this hereditary disorder.

Our earlier application, Serial No. 484,816, discloses partial-length cDNA clones and their use by which only up to 75% of the PKU families in the population can take advantage of the genetic analysis. It is highly desirable to provide for prenatal diagnosis and carrier detection of the genetic disorder for most PKU families, and the present invention is so directed.

The Phenylalanine Hydroxylasing System

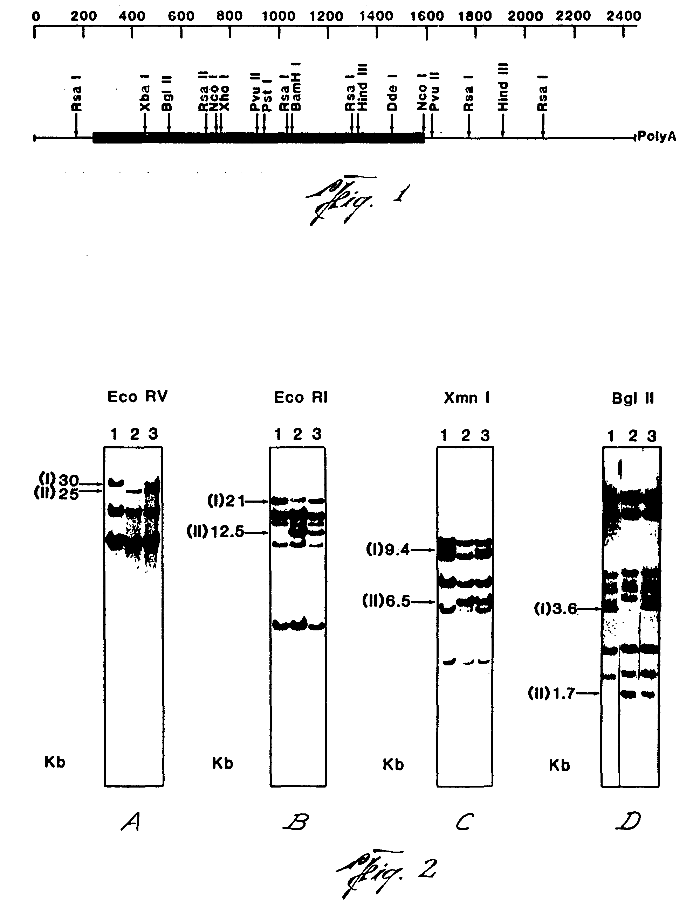

The mammalian phenylalanine hydroxylasing system is complex: it involves several other enzymes and cofactors in addition to phenylalanine hydroxylase itself (Fig. 1). In the presence of molecular oxygen and the active cofactor tetrahydrobiopterin, the enzyme oxidizes phenylalanine to tyrosine and simultaneously converts the cofactor into a quinonoid dihydrobiopterin form (Kaufman, S., 1964, J. Biol. Chem. 248, 540). The quinonoid dihydrobiopterin is subsequently reduced to the tetrahydrobiopterin form by dihydropteridine reductase in the presence of NADPH (Kaufman, S., 1976, In: Advances in Neurochemistry, Vol. 2, pp. 1-132, Agranoff, B.W. and Aprison, M.H., eds., Plenum Press, New York and London). Thus, the pterin cofactor functions catalytically during phenylalanine hydroxylation. Dihydrofolate reductase is required initially to convert the naturally occurring 7, 8-dihydrobiopterin into its active tetrahydro-form (Kaufman, S., 1962, In: Oxygeneses [O. Hayashi, ed.] p. 129, New York, Academic Press). This enzyme, however, is no longer a requirement after the initial reduction has occurred because the pterin cofactor recycles only between the tetrahydro-and quinonoid dihydro-forms (Kaufman, S., 1963, Proc. Natl. Acad. Sci. USA 50, 1085).

The entire chain of reactions occurs in the liver which is the only tissue to support significant phenylalanine hydroxylating activity in man.

<u>Heterogenous Phenotypes of Phenylalanine Hydroxylase Deficiency</u>

Since phenylalanine hydroxylation is such a complex system, it is conceivable that hyperphenylalaninemia may also result from deficiency in the other enzymatic activities or cofactors. Indeed variant forms of phenylketonuria have been described recently in which a deficiency in the enzyme dihydropteridine reductase has been demonstrated (Bartholome, K., 1974, Lancet 2:580; Smith <u>et al</u>, 1975, Arch. Dis. Child. 50, 864-870; Kaufman <u>et al</u>, 1975a, New Engl. J. Med. 293, 785-790; Kaufman, <u>et al</u>, 1975b, Pediat. Res. 9, 632-634). The PKU phenotype can also be manifested as the result of dihydrofolate reductase deficiency (Niederwieser, A., 1979, In: Folic Acid in Neurology, Psychiatry and Internal Medicine [Botez, M.I., Reynolds, E.H., eds.] pp. 349-384, New York, Ravin; Danks <u>et al</u>, 1979, Pediatr. Res. 13, 1150-1155) and homeostasis of the tetrahydrobiopterin coenzyme (Danks, D.M., 1978, J. Inherited Met. Dis. 1, 47-48). Nevertheless these types of "atypical phenylketonuria" constitutes a relatively minor proportion of phenylketonuric patients. A vast majority of them still lack phenylalanine hyaroxylase enzymatic activity in the liver biopsies, and hydroxylation of phenylalanine does not occur even in the presence of added tetrahydropterin cofactor (Grimm <u>et al</u>, 1975, Clin. Chim. Acta 58, 17-21).

Phenylalanine hydroxylase deficiency, however, is a heterogeneous state, since patients with this condition can vary from severe (classical PKU) to moderate (hyperphenylalanemia). Phenylalanine hydroxylase has been purified from livers of rat, monkey and human (Kaufman, S. and Fisher, D.B., 1970, J. Biol. Chem. 245, 4745-4750; Gillam et al, 1974, Biochem. J. 139, 731-739; Choo, K.H. and Cotton, R.G.H., 1979, Biochem. J. 17, 921-946; Woo et al, 1974, Biochem. J. 139, 741-749; Choo et al, 1979a, Biochem. J. 181, 285-294; Choo et al, 1979b, J. Inherited Metab. Dis. 2, 79-84). It is a multimeric enzyme with a subunit molecular weight of about 50,000 daltons. Recent evidence has shown that the subunits are either identical or one being a proteolytic product of the other (Choo et al, 1979a, Biochem. J. 181, 285-294; Choo et al, 1979b, J. Inherited Metab. Dis. 2, 79-84). Phenylalanine hydroxylase from patients with classical phenylketonuria has been shown to be a structurally altered form of the enzyme with less than 1% of the normal activity (Friedman et al, 1973, Proc. Natl. Acad. Sci. USA 70, 552-556), indicating phenylketonuria may be the direct result of a mutation in the phenylalanine hydroxylase gene itself. Furthermore, analysis of phenylalanine hydroxylase activity in liver biopsies of patients with "hyperphenylalaninemia" has shown that they contained about 5% of the normal activity level, with a range of 1 to 35% (Kaufman et al, 1975a, New Engl. J. Med. 293, 785-790; Kaufman, et al, 1975b, Pediat. Res. 9, 632-634; Bartholome et al, 1975, Pediat. Res. 9, 899-903). It has subsequently been demonstrated that the low enzymatic activities in cases of hyperphenylalaninemia was not due to the presence of 5% of the normal enzyme but rather to the presence of an altered hydroxylase with kinetic properties distinct from both the normal enzyme and the enzyme from patients with classical PKU (Kaufman, S., 1976, In: Advances in Neurochemistry, Vol. 2, pp. 1-132, Agranoff, B.W. and Aprison, M.H., eds., Plenum Press, New York and London; Kaufman, S. and Milstein, S., 1977, Ann. Clin. Lab. Sci. 2, 178-185). Together, these studies clearly indicate the presence of multiple phenotypes of phenylalanine hydroxylase deficiency, which could be caused by mutations at various locations in the phenylalanine hydroxylase gene.

Phenylalanine hydroxylase (PAH) cDNA clones have been isolated from rat and human liver cDNA libraries. In our earlier application, Serial No. 484,816, and in Nature, Vol. 306, No. 5939, pp. 151-155, 10 November, 1983, we disclosed the use of human PAH cDNA clones representing the 3' half of the mRNA to identify restriction fragment length polymorphisms in the PAH locus in man. The polymorphisms were found using the restriction enzymes MspI, SphI and HindIII, and have been applied to trace the transmission of the mutant PAH genes in informative PKU families with one or more affected children. These experiments have demonstrated that the mutant PAH genes segregated concordantly with the disease state. The frequencies of the restriction site polymorphisms in the PAH gene detected by these partial-length cDNA clones are such that, even under ideal conditions, only 75% of the PKU families in the population can take advantage of the genetic analysis. It would be highly advantageous to identify additional restriction fragment length polymorphisms in the PAH locus, thereby providing for haplotype analysis of most PKU families for prenatal diagnosis and carrier detection of the genetic disorder. In the present invention, this is accomplished by the isolation and use of a full-length human PAH cDNA clone.

Determination of the Chromosomal Location of the PKU Locus

The PKU locus in man has been studied by linkage analysis with other polymorphic gene loci in PKU families with inconclusive results. The possible assignment of the PKU locus on chromosome 1 was suggested by moderate linkage data with the phosphoglucomutase locus PGM-1 (1p3) (Berg, et al, A Linkage Study of Phenylketonuria, Clin. Genet. 6:147-152, 1974) and the amylase loci Amy-1 and Amy-2 (1p1) (Kamaryt, et al, PKU Locus, Genetic Linkage with Human Amylase (Amy) Loci and Assignment to Linkage Group 1, J Human Genet. 43:205-210, 1978). More recently, linkage studies between the PKU locus and 15 chromosome markers including PGM-1, Amy-1 and Amy-2, using improved methods for heterozygote determination among siblings in the PKU families, have been performed. These experiments, however, failed to establish genetic linkage between the PKU locus and any of the markers and are in disagreement with the previous assignment (Paul, et al, Linkage Analysis Using Heterozygote Detection in Phenylketonuria, Clin. Genet. 16:217-232, 1979).

The use of recombinant DNA probes in conjunction with human/rodent cell hybrid panels to assign specific genes in the human genome is a powerful tool in the construction of the human gene map. Chromosomal assignments for genetic loci can be made using cloned genes as probes in molecular hybridization to genomic DNAs isolated from human/ rodent cell hybrids that contain different assortments of human chromosomes. The human phenylalanine hydroxylase cDNA clones were used to detect the presence of the human phenylalanine hydroxylase gene in a clonal human/mouse hybrid cell panel by molecular hybridization. Comparison of these data with the presence of various human chromosomes in each of the hybrid cell lines has shown that the human phenylalanine hydroxylase gene is on chromosome 12.

The human phenylalanine hydroxylase cDNA clone has been used as a hybridization probe to detect the existence of restriction fragment length polymorphisms in the phenylalanine hydroxylase locus (Woo, et al, Cloned Human Phenylalanine Hydroxylase Gene permits Prenatal Diagnosis and Carrier Detection of Classical Phenylketonuria, Nature 306:151-155, 1983). The DNA of family members from families with one or more PKU children plus unaffected siblings has been analyzed. In all families studied, complete segregation concordance between the mutant phenylalanine hydroxylase gene and the disease phenotype has been observed. Furthermore, the allelic segregation between the probands and the unaffected siblings was totally discordant. These results strongly suggest that PKU is the result of mutational events in the phenylalanine hydroxylase gene itself and is not caused by some other transregulatory mechanisms. Since the phenylalanine hydroxylase gene has been assigned to human chromosome 12, the PKU locus in man is also on chromosome 12. Additionally, the existence of multiple restriction fragment length polymorphisms in the phenylalanine hydroxylase locus indicates that the full-length human cDNA clone can be used as a polymorphic marker for chromosome 12 and thus be employed in linkage analysis between the PKU and other neighboring human gene loci.

SUMMARY

Accordingly, the present invention is directed to isolated full-length human phenylalanine hydroxylase cDNA clones, methods of their production, and the use of these clones in the prenatal diagnosis of the human genetic disorder, PKU, and in detecting carriers of the phenylalanine hydroxylase deficiency trait. Using the full-length cDNA clone as a hybridization probe to analyze human genomic DNA, a total of 8 restriction enzymes that yield polymorphic patterns from the corresponding chromosomal gene are identified.

The frequencies of the restriction site polymorphisms in the human phenylalanine hydroxylase locus are such that the percent heterozygosity among Caucasians is estimated to be well over 95%, suggesting that most of the families with history of classical phenylketonuria can take advantage of the genetic analysis for prenatal diagnosis and carrier detection of the hereditary disorder, as previously mentioned.

This invention is also directed to a method of using the human phenylalanine hydroxylase cDNA clones to determine the chromosomal location of the PKU locus in man, and the isolation of the corresponding human chromosomal gene.

This invention is also directed to identifying PKU effected fetus, and PKU trait carriers without a proband.

Advantageously, standard, approved, recombinant DNA technology is employed and the components, such as the plasmids, etc., are readily available on the market. Details of the invention are set forth below.

BRIEF DESCRIPTION OF THE DRAWINGS

Figure 1 is a restriction map of phPAH247 which is a full-length phenylalanine hydroxylase cDNA clone. It consists of about 2400 nucleotides inserted into the EcoRI site of pBR322. The coding region is highlighted by the thick line and untranslated regions are represented by the thin lines.

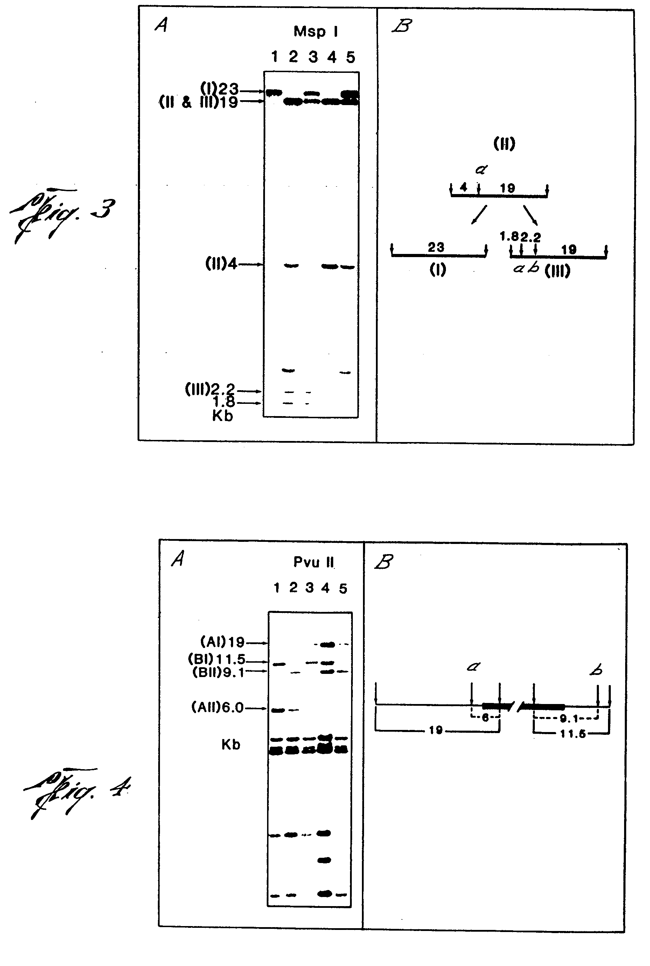

Figure 2 comprises radioautograms of the human phenylalanine hydroxylase gene after analysis by Southern hybridization using the restriction enzymes that each yielded 2 polymorphic DNA fragments. The DNA samples were obtained from lymphocytes of random normal Caucasians. The enzymes used for DNA digestion in Panels A-D are EcoRV, EcoRI, XmnI, and BglII, respectively. The Roman numerals are allele designations according to hybridized fragment sizes.

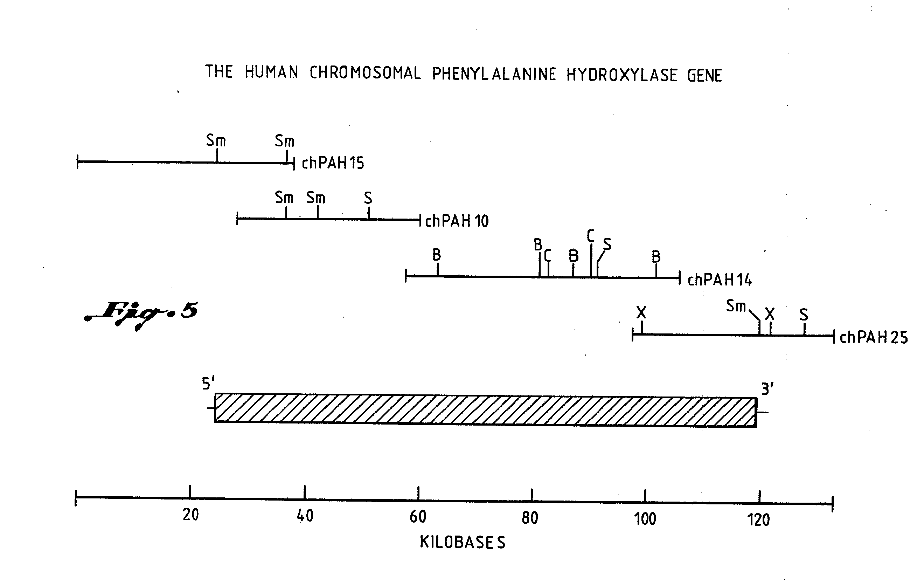

Figure 3 is a Southern blot analysis of the human phenylalanine hydroxylase gene by the enzyme MspI. The left-hand panel is a radioautogram of 5 individuals' DNAs after hybridization with phPAH247 showing 5 different patterns. The right-hand panel is a hypothetical schematic representation illustrating the possible origin of the restriction fragment length polymorphism with the MspI sites designated by the arrows.

Figure 4 is a Southern blot analysis of the human PAH gene by the enzyme PvuII. The left-hand panel is a radioautogram of 5 individuals' DNAs after hybridization with phPAH247. The right-hand panel is a schematic diagram of a hypothetical illustration on the organization of the polymorphic fragments. The heavy dark lines represent the structural portions in the gene which hybridized to the cDNA probe. The polymorphic PvuII sites are designated A and B.

Figure 5 is a schematic representation of the human DNA inserts in four recombinant cosmid clones containing overlapping fragments of the human chromosomal phenylalanine hydroxylase gene. The approximate domain of the gene is shown as the latched box in the composite profile underneath. Symbols used for the restriction sites in the clones are Sm, SmaI; S, SalI; B, BamHI; C, ClaI and X, XhoI.

## PRESENTLY PREFERRED EMBODIMENTS

### Molecular and Biological Basis of Classical Phenylketonuria

The following organisms are available from permanent collection of the American Type Culture Collection, 12301 Parklawn Drive, Rockville, Maryland 20852, U.S.A.

| | |
|---|---|
| ATCC 39644 | E. Coli RR1 (phPAH247) |
| ATCC 39660 | E. Coli ED8767 (chPAH15) |
| ATCC 39659 | E. Coli ED8767 (chPAH10) |
| ATCC 39661 | E. Coli ED8767 (chPAH14) |
| ATCC 39658 | E. Coli ED8767 (chPAH25) |

The deposits are available to the public upon a grant of a patent to the Assignee, Howard Hughes Medical Institute, disclosing them. It should be understood, however, that the availability of a deposit does not constitute a license to practice the subject invention in derogation of patent rights granted by governmental action.

Restriction fragment length polymorphism (RFLP) is an example of human variation analyzed at the genotypic level. These polymorphisms can be used much like phenotypic polymorphisms, i.e. electrophoretic variants of proteins, to describe the variety within and between populations, to do linkage analysis between an RFLP and a trait of unknown location and to do kindred analysis of traits co-segregating or tightly linked to the probe used to identify the RFLP. We have identified many RFLP's in the phenylalanine hydroxylase locus by using a full-length cDNA as the hybridization probe. These polymorphisms are frequent and suggest a high level of heterozygosity. In fact, each of the 19 individuals analyzed was heterozygous for one or more of the RFLPs.

Since phenylalanine hydroxylase is found mainly in the liver, it does not easily lend itself to rigorous characterization. In fact, many questions remain unanswered as to the biochemistry of the enzyme. Phenylketonuria has been well characterized clinically, but the defect in phenylalanine hydroxylase has not been identified. The molecular approach to the study of this inborn error in metabolism has been initiated with the cloning of the cDNA for phenylalanine hydroxylase. Restriction fragment length polymorphism in the chromosomal phenylalanine hydroxylase gene is a useful tool in studying PKU. First of all, the incidence of haplotypes, as defined by the RFLPs, associated with normal and PKU genes can be defined for different populations. This type of information has been useful in identifying the many different mutations of β-Thalassemia, most of which are associated with specific haplotypes.

It is possible to distinguish the normal and mutant phenylalanine hydroxylase genes in families with one or more affected children by coupling a unique haplotype with each PKU gene in each family. The segregation of haplotypes with the PKU trait in families tested has shown that the polymorphisms can be used for prenatal diagnosis in future pregnancies and for PKU heterozygote detection among siblings in families with one or more PKU children. This analysis relies on both parents being haplotype heterozygotes so that their two phenylalanine hydroxylase genes can be distinguished. The additional polymorphisms set forth are useful in that it expands the percent heterozygosity in the population. This means that most PKU families will be informative for the polymorphisms and will be able to take advantage of prenatal diagnosis and carrier detection.

## MATERIALS AND METHODS

### The Recombinant Plasmid phPAH247

Plasmid phPAH 247 is a recombinant containing a full-length human cDNA for phenylalanine hydroxylase, which was identified from a human liver cDNA library subsequently described in detail. The cDNA insert was subcloned into the EcoRI site of pBR322 and isolated by preparative digestion of phPAH247 DNA with EcoRI followed by electrophoresis in low-melting agarose gels. The DNA was extracted and nick-translated to a specific activity of 2-3 X 108dpm/μg by standard procedures.

### The Recombinant Cosmid Clones chPAH15, chPAH10, chPAH14 and chPAH25

Cosmid clones chPAH15, chPAH10, chPAH14 and chPAH 25 are recombinants containing various portions of the normal human chromosomal phenylalanine hydroxylase gene, which were identified from a genomic DNA library constructed using the cosmid vector pCV107 subsequently described in detail. Southern hybridization experiments showed that clones chPAH25 and chPAH10 contained the 5' portion, chPAH14 contained the middle portion and chPAH25 contained the 3' portion of the gene. All together the four clones represent about 130 kb of human genomic DNA containing the PAH locus.

### Construction of a Human Liver cDNA Library

λgt11. A post-mortem human liver specimen was kindly provided by Dr. Bill Williams (University of Texas Medical School at Houston). Human liver total polyA-containing RNA and cDNA were prepared as previously described in Chandrda, et al, (1983) Proc. Natl. Acad. Sci., USA, 80, 1845-1848. The cDNA preparation was sedimented through an alkaline sucrose gradient (Monahan, J. J., et al, 1976 Biochemistry, 15, 223-233) and only fractions containing cDNA species of greater than 1,500 nucleotides were pooled. The cDNA preparation was made double-stranded using reverse transcriptase (Chandrda, T., et al, 1983 Proc. Natl. Acad. Sci., USA, 80, 1845-1848) and after S1 nuclease treatment, the residual staggered ends in the DNA preparation were filled-in using the Klenow fragment of DNA polymerase in the presence of all 4 deoxyribonucleoside triphosphates (Maniatis, T., et al, 1982 in Molecular Cloning: A Laboratory Manual eds. Maniatis, T., et al, [Cold Spring Harbor Laboratory] pp. 97-149). The blunt-ended cDNA was treated with EcoRI methylase prior to ligation with phosphorylated EcoRI linkers using T4 DNA ligase (Maniatis, T., et al, 1982 in Molecular Cloning: A Laboratory Manual eds. Maniatis, T., et al, [Cold Spring Harbor Laboratory] pp. 97-149). The ligated DNA preparation was exhaustively digested with EcoRI and double-stranded DNAs greater than 1,500 base pairs in length were obtained by sucrose density gradient centrifugation under non-denaturing conditions, which also removed all of the excess EcoRI linkers (Maniatis, T., et al, 1982 in Molecular Cloning: A Laboratory Manual eds. Maniatis, T., et al, [Cold Spring Harbor Laboratory] p. 396). The phage vector λgt11 and E. Coli host strains Y1088 and Y1090 (Young, R. and Davis, R. 1983 Proc. Natl. Acad. Sci. USA, 80, 1194-1198) were kindly provided by Drs. Richard Young and Ron Davis at Stanford. Native λgt11 DNA was ligated into conca-temers, digested to completion with EcoRI and the 5' terminal phosphates were removed by bacterial alkaline phosphatase. The pooled human liver cDNA was ligated with the vector DNA, packaged in vitro, and used for infection of E. Coli Y1088 as described in Young, R. and Davis, R. 1983 Proc. Natl. Acad. Sci. USA, 80, 1194-1198. Approximately 14 million primary phage plaques were generated in this library. Greater than 90% of these are recombinants containing human DNA inserts as suggested by their lack of β-galactosidase activity (Young, R. and Davis, R. 1983 Science 222, 778-782) and characterization of 20 randomly selected cDNA clones by EcoRI digestion followed by agarose gel electrophoresis. Phage particles were pooled from the plates and purified using isopycnic CsCl gradients and stored in SM buffer (Maniatis, T., et al, 1982 in Molecular Cloning: A Laboratory Manual, eds. Maniatis, T., et al, [Cold Spring Harbor Laboratory] pp. 75-97).

Screening of the λgt11 cDNA Library for Human Phenylalanine Hydroxylase cDNA Clones

The recombinant λgt11 cDNA library was screened using a previously cloned partial-length human phenylalanine hydroxylase cDNA by the method of Woo (Methods in Enzymology, 68, p. 389, 1979). The E. Coli host cells were grown at 37°C in L-broth and infected with the recombinant λ phage library. The infected cells were mixed with 0.7% agar in T-broth and plated on 1% agar in L-broth. Both the T-soft agar and L-hard agar were supplemented with 10 mm $MgSO_4$. The plates were incubated at 37° to allow the formation of phage plaques. Sterile nitrocellulose filters were soaked in a suspension of E. Coli host cells in T-broth containing 10 mm $MgSO_4$. The filters, after coating with E. Coli cells, were blotted dry on sterile Whatman 3MM papers in a bioguard hood. The dried nitrocellulose filters were layered onto the phage-containing agar plates and stored in a refrigerator for about 30 minutes to allow the transfer of phage particles. The filters were subsequently removed from the phage-containing agar plate and transferred onto fresh L-agar plates supplemented with 10 mm $MgSO_4$, with the side that has come in contact with the phage particles facing the air. The fresh petri dishes containing the nitrocellulose filters were incubated at 37°C until the bacteria coating had grown to establish a lawn on the nitrocellulose filters and phage particles transferred onto the nitrocellulose filters from the original plate had formed plaques on the bacterial lawn. The nitrocellulose filters were removed from the agar dishes and subjected to treatment with the following solutions:

(1) 0.5 N NaOH/1.5 N NaCl; (2) 1.0 M Tris-HCl, pH 7.4; and (3) 0.5 M Tris-HCl, pH 7.4/1.5 M NaCl. The filters were air-dried, baked in a 68°C oven for two hours and coated at 68°C for several hours with a solution containing 6 xSSC and 0.2% each of bovine serum albumin, ficoll and polyvinylpyrrolidone according to the procedure of Denhardt (Biochem. Biophys, Res. Commun., 23, 641, 1966) The coated filters were then subjected to hybridization with $^{32}$P-labeled phPH72 DNA in Denhardt's solution containing 0.5% Sodium dodecyl sulfate. Hybridization was carried out at 68°C overnight with $10^{6}$cpm of hybridization probe per filter. The nitrocellulose filters were washed at 68°C with three changes of 1 xSSC containing 0.5% SDS, blotted dry at room temperature and subjected to radiotography at -20°C using Dupont Cronex 4 X-ray films in the presence of intensifying screens. Recombinant phage plaques identified by the screening procedure were picked from the original master plate and subjected to the screening procedure for a second time. After secondary screening single isolates of recombinant phage plaques yielding hybridization signals were obtained. Recombinant phages were then cultured and the DNA isolated by standard procedures. The recombinant λDNAs were digested with EcoRI and the sizes of the inserted human DNA fragments were analyzed by agarose gel electrophoresis followed by EtBr staining. The clone with the largest DNA insert was designated phPAH247 which was chosen for further analysis.

Southern Blotting and Hybridization

Total human genomic DNA was purified from peripheral leukocytes isolated as buffy coats from whole blood as previously described in Woo, et al, Nature 309, 151-155, 1983. Five micrograms of genomic DNA was digested to completion with restriction enzymes in appropriate buffers as recommended by the manufacturer. Electrophoresis and blotting to nitrocellulose paper were done as described in Southern, J., Molec. Biol., 98, 503-517, 1975. Prehybridization and hybridization were done at 42°C in 45% formamide, 5x Denhardt's solution, 4xSSC, 0.1M $NaPO_4$ ph 6.5, 0.1 %SDS, 0.1 % sodium pyrophosphate, 250 µg/ml sheared, denatured Herring Sperm DNA. Hybridization was done in the presence of 10% Dextran Sulfate and 2 X $10^6$cpm/ml $^{32}$P-labeled probe insert. Nitrocellulose papers were washed twice in 2 xSSC, 0.5% SDS for 15' at room temperature, and in 0.1 xSSC, 0.1% SDS for 2 hours at 68°C, followed by autoradiography for 1-5 days.

Chromosomal Assignment of the PKU Locus

Hybrid Cell Lines and DNA Isolation

Hybrid parental cell lines were made by fusing mouse A9 cells with human GM144 cells (AHA), human GM589 cells (BDA), and human WI-38 cells (WA); or between mouse L-cells and human GM126 cells (41pt and FRY4). Subclones of the original hybrid cell lines were isolated by dilution plating. Mouse-human hybrid cells were cultivated without antibiotics in α-minimal essential medium, 10% heatinactivated fetal bovine serume, in roller bottles. Cytogenetic and isozyme samples were taken concurrently with DNA isolation. Monolayers were twice washed with saline and once with TNE (50 mM Tris-HCl, pH 8, 10 mM NaCl, 10 mM EDTA) at 4°C. TNE supplemented with 0.5% w/v SDS (BRL) and proteinase K (100 µ/ml) was added followed by 37°C overnight incubation on a roller mill. DNA was extracted twice with redistilled phenol, twice with chloroform-isoamyl alcohol (24:1), concentrated in dialysis bags against PEG 8000 (Sigma, St. Louis, Mo.), and exhaustively dialyzed against TE (10 mM Tris-HCl, pH 8, 1 mM EDTA) at 4°C.

Karyotype and Isozyme Analysis of Hybrid Cell Line

Genetic analysis of cell hybrids included G-banding of 25-50 metaphases and isozyme analysis. The isozyme constitution of each hybrid cell line was determined by starch gel or cellulose acetate electrophoresis and histochemical staining for the following loci: chromosome 1 (enolase-1, E.C.4.2.1.11; phosphoglucomutase-1, E.C.2.7.5.1; and peptidase-C, E.C.3.4.11*); chromosome 2 (malate dehydrogenase-1, E.C.1.1.137; and isocitrate dehydrogenase-1, E.C.1.1.1.42); chromosome 3 (acylase-1, E.C.3.5.1.14); chromosome 4 (peptidase-S, E.C.3.4.11I; and phosphoglucomutase-2, E.C. 2.7.5.1); chromosome 5 (hexosaminidase B, E.C.3.2.1.30); chromosome 6 (glyoxylase-1, E.C.4.4.1.5; and malic enzyme-1, E.C. 1.1.1.40); chromosome 7 (β-glucuronidase, E.C.3.2.1.31; and uridine phosphorylase, E.C.2.4.2.3); chromosome 8 (glutathione reductase, E.C.1.6.4.2); chromosome 9 (adenylate kinase-1, E.C.2.7.4.3; and aconitase-S, E.C.4.2.1.3); chromosome 10 (adenosine kinase-1, E.C. 2.7.1.20; and glutamate oxaloacetate transaminase, E.C.2.6.1.1); chromosome 11 (lactate dehydrogenase-A, E.C.1.1.1.27); chromosome 12 (triose phosphate isomerase, E.C.5.3.1.1.; peptidase-B, E.C.3.4.11*; and lactate dehydrogenase-B, E.C.1.1.1.27); chromosome 13 (esterase-10, E.C.3.1.1.1.); chromosome 14 (nucleoside phosphorylase, E.C.2.4.2.1); chromosome 15 (mannose phosphate isomerase, E.C.5.3.1.8; pyruvate kinase-3, E.C.2.7.1.40; and hexosaminidase-A, E.C.3.2.1.30); chromosome 16 (adenosine phosphoribosyl transferase, E.C.2.4.2.7); chromosome 17 (galactokinase, E.C.2.7.1.6); chromosome 18 (peptidase-A, E.C.3.4.1*); chromosome 19 (glucose phosphate isomerase, E.C.5.3.1.9); chromosome 20 (adenosine deaminase, E.C.3.5.4.4.); chromosome 21 (superoxide dismutase-1, E.C.1.15.1.1); chromosome 22 (aconitase-M, E.C.3.1.6.1); and the X chromosome (phosphoglycerate kinase, E.C.2.7.2.3; hypoxanthine-guanine phosphoribosyl transferase, E.C.2.4.2.8; and glucose-6-phosphate dehydrogenase, E.C.1.1.1.49).

Southern Blotting Analysis

About 5-10 µg of DNA were digested to comple-tion with BamH1 at 2 U/µg DNA followed by electrophoresis in 1% agarose gels. DNA was transferred to a nitrocellulose filter according to the method of Southern (Southern, EM: Detection of Specific Sequences Among DNA Fragments Separated by Gel Electrophoresis., J. Mol. Biol. 98:503-517, 1975) and allowed to hybridize with the inserted Pst I fragment of phPH72 DNA after labeling with $^{32}$P by nick-translation (Maniatis, T., et al, Nucleotide Sequence of the Rightward Operator of Phage λ, Proc. Natl. Acad. Sci. USA 72:1184-1188, 1975). Conditions of hybridization and washing were as those reported by Wahl, et al (Wahl, et al, Efficient Transfer of Large DNA Fragments from Agarose Gels to Diazobenzyloxymethol-Paper and Rapid Hybridization by Using Dextran Sulfate, Proc. Natl. Acad. Sci. USA 76:3683-3687, 1979). Hybridization bands were detected by autoradiography using Fuji Medical X-ray films with intensifying screens at -70° for up to three days.

RESULTS

A Full-Length Human Phenylalanine Hydroxylase cDNA Clone

phPAH247 is a recombinant plasmid which contains the full-length human cDNA for phenylalanine hydroxylase inserted into the EcoRI site of pBR322. The cDNA is about 2.4kb in length and includes the entire coding region, 5' and 3' untranslated sequence, and a stretch of polyA's as shown in Figure 1. Southern blot analysis of total human genomic DNA digested with a number of restriction enzymes using phPAH 247 as a hybridization probe indicates that the chromosomal phenylalanine hydroxylase gene is a large gene containing multiple intervening sequences. The cloning and preliminary characterization of the chromosomal phenylalanine hydroxylase gene has shown that all fragments detected by Southern analysis of genomic DNA arise from a single copy gene which is greater than 80 kb in length.

## Multiple Restriction Fragment Length Polymorphisms in the Human PAH Locus

High molecular weight genomic DNA isolated from peripheral leukocytes of 19 unrelated normal Caucasian individuals was digested with a number of restriction enzymes followed by Southern blot analysis using phPAH247 as the hybridization probe. The list of enzymes used for the analysis includes the following: AvaII, BamHI, BclI, BglII, BstNI, EcoRI, EcoRV, HaeIII, HincII, HindIII, KpnI, MspI, PstI, PvuII, Sau96AI, ScrFI, SphI, SstII, TaqI, SbaI, and XmnI. In addition to SphI, HindIII and MspI reported previously, five additional enzymes that yielded restriction fragment length polymorphisms were observed:

EcoRV: phPAH247 hybridizes to three invariant fragments and a fourth allelic fragment of either 30 kb or 25 kb. Individuals homozygous for the 30 kb fragment (Figure 2A; lane 1) lack the polymorphic EcoRV restriction site, and those homozygous for the 25 kb fragment (Figure 2A; lane 2) have the polymorphic EcoRV site in each of their phenylalanine hydroxylase genes. Heterozygotes for the restriction site in their chromosomes have also been detected (Figure 2A; lane 3).

EcoRI: Among the multiple EcoRI restriction fragments containing various portions of the phenylalanine hydroxylase gene, the 21 kb fragment contains a polymorphic EcoRI restriction site. The absence of the site in both copies of the gene results in a pattern shown in Figure 2B, lane 1. The presence of the site in the 21 kb fragments results in its cleavage into a 12.5 kb hybridizable fragment, and individuals homozygous for this site have a pattern as shown in Figure 2B, lane 2. The pattern is complicated by the presence of an additional invariant but much fainter band which comigrates with the polymorphic 21 kb fragment as shown in Figure 2B, lane 2. However, individuals heterozygous for the polymorphic EcoRI site (Figure 2B, lane 3) have the 12.5 and 21 kb bands of almost equal intensity and are readily distinguishable from those homozygous for the 12.5 kb fragment as shown in Figure 2B, lane 2.

XmnI: A two allelic system was also detected by this enzyme. Restriction site polymorphism results in homozygous individuals for either a 9.4 kb fragment shown in Figure 2C, lane 1, or a 6.5 kb fragment shown in Figure 2C, lane 2. Heterozygous individuals for the two fragments have also been identified as shown in Figure 2C, lane 3.

BglII: One BglII site is polymorphic in the phenylalanine hydroxylase gene and results in restriction fragments of either 3.6 or 1.7 kb in length. Individuals homozygous for the 3.6 kb fragment (Figure 2D, lane 1) or for the 1.7 kb fragment (Figure 2D, lane 2) and those heterozygous for the two fragments (Figure 2D, lane 3) have been readily detected in the panel of random individuals.

MspI: The MspI polymorphism in the phenylalanine hydroxylase gene detected previously with the partial-length human cDNA clones consists of two alleles: 23 and 19 kb. Hybridization of MspI-digested genomic DNA of the 19 kb genotype with phPAH247 results in the identification of the 4 kb DNA fragments not detected previously as shown in Figure 3, lanes 2, 4, 5. There is an additional MspI polymorphic site within the 4.0 kb fragment in that it could be digested into 2.2 and 1.8 kb DNA fragments in some individuals as shown in Figure 3, lanes 2, 3. As depicted schematically in Figure 3, the 23 kb allele (I) and the (19+4) kb allele (II) are created by the absence or presence of an internal MspI site, respectively. The additional cDNA sequence in phPAH247 has allowed the identification of a second polymorphic MspI site internal to the 4 kb fragment (allele III).

PvuII: Southern analysis of DNA digested with PvuII shows four fragments of variable length created by two polymorphic PvuII restriction sites. The four fragments segregate as two separate sets of alleles. The two polymorphic sites, designated A and B, are also illustrated schematically in Figure 4. The presence of site A results in cleavage of a 19 kb fragment into two fragments, of which only the 6 kb fragment hybridizes to phPAH247. The second site B results in the cleavage of the 11.5 kb fragment into a 9.1 kb hybridizable fragment. There can be a total of ten possible haplotype combinations between the two sets of alleles and the Southern blot in Figure 4 shows 5 such cleavage patterns of PvuII-digested DNA. Individuals homozygous for the AII/BI, AI/BI, and AI/BII alleles are shown in lanes 1, 3, and 5, respectively, of Figure 4. A heterozygote for both sets of alleles is shown in lane 2 and an individual homozygous for the AI allele but heterozygous for site B polymorphism is shown in lane 4 of Figure 4.

Allele Frequencies and Percent Heterozygosity

It is important to determine the degree of restriction fragment length polymorphism in the human phenylalanine hydroxylase gene to estimate the percentage of PKU families in the population that can take advantage of the genetic analysis for prenatal diagnosis and carrier detection. The frequency of each allele, as defined by the presence or absence of a particular polymorphic restriction site, is listed in Table 1. These frequencies were calculated from a sample size of 38 chromosomes in a panel of 19 normal Caucasian individuals. Some of the restriction site polymorphism reaches 0.5 in frequency and will be very useful for genetic analysis. For instance, the EcoRV polymorphism alone gives a percent heterozygosity of almost 50%. The definition of a PAH gene by haplotype is particularly powerful for identification of individual genes in PKU families. Not taking into consideration the possibility of linkage disequilibrium between the polymorphic restriction sites, there could be a total of over 1,000 possible haplotypes of the

4. Transfer the DNA fragments from the agarose gel to a nitrocellulose filter or equivalent materials by Southern blotting.

5. Pre-incubate the filter at 42°C for several hours in a prehybridization solution, followed by hybridization overnight at 42°C with $2 \times 10^6$ cpm/ml of $^{32}$P-labeled PAH DNA probe in the presence of 10% dextran sulfate as described in the "Southern Blotting and Hybridization" section of this application.

6. Wash the filter at room temperature twice with 2xSSC containing 0.5% SDS for 15', followed by washing twice at 68°C with 0.1xSSC containing 0.1% SDS for 2 hours. Blot dry the filter and subject it to radioautography for 1-5 days.

7. Develop the X-ray film and determine the RFLP haplotypes of the 4 PAH genes in the family. Identify the restriction enzymes that yielded polymorphic patterns that would distinguish the mutant PAH genes from the normal ones.

8. Obtain fetal cell samples by amniocentesis followed by culturing of the amniotic cells. These are routine procedures employed in major hospitals for prenatal diagnosis of a variety of genetic abnormalities. Alternatively fetal chorionic villi can also be used as the source of· fetal cells when the procedure becomes widely adopted.

9. Isolate fetal genomic DNA from the amniotic cells or chorionic villi and analyze the fetal DNA sample by Southern hybridization using the previously determined restriction enzyme(s).

10. Compare the polymorphic patterns generated by the fetal DNA sample to those of the proband and the parental DNA samples. Determine whether the fetus has inherited two copies of the mutant PAH genes from both parents.

11. The same analysis using steps 1-7 also provides the means for identification of trait carriers in uneffected siblings in PKU families.

-26-

## Cloning and Characterization of the Normal and Mutant Chromosomal Phenylalanine Hydroxylase Genes

Genomic DNA's isolated from normal and affected individuals can be used for construction of chromosomal DNA libraries using either λ or cosmid cloning vectors (Lawn, et al, Cell 15, 1157, 1978; Hohn and Collins, Gene 11, 291, 1980), from which DNA clones containing various chromosomal genes can be identified by screening with the corresponding cloned cDNA probes. High molecular weight genomic DNA was isolated using standard procedures from peripheral lymphocytes of a Caucasian individual with two normal phenylalanine hydroxylase genes as determined by RFLP haplotype analysis. The DNA was digested partially with MboI and sedimented through a 1.25-5.0 M NaCl gradient at 20° for three hours at 39,000 rpm in a SW40 rotor. The salt gradient was fractionated and the size range of DNA contained in each fraction was determined by agarose gel electrophoresis. Those fractions containing DNA fragments of 40-50 kb in length were pooled, precipitated with ethanol and redissolved in a small volume of water. The cosmid vector pCV107 and E. Coli ED8767 host were kindly provided by Dr. Y. W. Kan at the University of California at San Francisco. pCV107 DNA was digested to completion with BamHI and 5'-phosphate groups were removed by treatment with bacterial alkaline phosphatase as described for the λgt11 vector in a previous section. The de-phosphorylated linear pCV107 DNA vector and the genomic DNA fractions were ligated using T4 DNA ligase at concentrations of 150 µg/ml and 75 µg/ml, respectively. The ligation mixture was then packed in vitro and used for transduction of E. Coli ED8767 cells as described in Lau and Kan, Proc. Natl. Acad. Sci. USA 80, 5225, 1983. The transduced E. Coli ED8767 cells were selected as colonies on L-agar plates containing 50 µg/ml of ampicillin. A genomic DNA library of about $5 \times 10^5$ independent recombinants was thus constructed. The library was then screened using cloned human phenylalanine hydroxylase cDNAs as the hybridization probe and a number of genomic DNA clones containing various portions of the human chromosomal phenylalanine hydroxylase gene were obtained. As shown in Figure
5, four of the genomic DNA clones, designated chPAH 15,
chPAH 10, chPAG 14 and chPAH 25, contain overlapping DNA
fragments encompassing approximately 130 kb of human genomic
DNA. Preliminary characterization of these genomic DNA
clones by Southern hybridization analysis has indicated that
the human chromosomal phenylalanine hydroxylase gene is
greater than 80 kb in length as shown in the latched box of
Figure 5.

The genomic DNA clones containing various portions
of the human chromosomal phenylalanine hydroxylase gene can
be further analyzed structurally by restriction mapping,
Southern hybridization and electronmicroscopic mapping using
procedures previously reported by our laboratory (Leicht et
al, Nature 297, 655, 1982). The molecular details of the
gene can be established by performing DNA sequence analysis.
DNA sequences intronic to the phenylalanine hydroxylase gene
can also be used as hybridization probes to detect the
existence of additional polymorphisms in the gene after the
identification and removal of repetitive sequences. The
same will also apply to DNAs that are adjacent to the
phenylalanine hydroxylase gene. Polymorphisms detected by
these additional chromosomal DNA probes will be equally
useful for prenatal diagnosis and carrier detection of
classical PKU in affected families.

Mutant phenylalanine hydroxylase genes can be
isolated from affected individuals using similar methods as
described above and can be characterized in detail by DNA
sequencing analysis. Comparison of DNA sequences between
the mutant and normal genes should reveal the molecular
basis of the genetic disease. If the basic genetic lesion
occurs at a restriction recognition sequence, the respective
mutant gene can be detected in affected individuals as well
as carriers by analyzing their respective genomic DNAs using
the particular restriction enzyme followed by Southern hybridization with the cloned phenylalanine hydroxylase DNA probe. An example of this method of analysis is the use of MstII in the identification of the mutant β-globin gene responsible for sickle cell anemia (Orkin et al, New Engl. J. Med. 307, 27, 1982). If the molecular lesion does not occur at a restriction recognition sequence, specific oligonucleotides of the normal and the mutant sequences can be synthesized chemically, labeled in vitro and used as hybridization probes to identify the mutant genes in affected individuals as well as carriers. This method of analysis was developed using sickle cell anemia as the prototype (Conners et al, Proc. Natl. Acad. Sci. USA 80, 278, 1983) and has been reported previously by us for analysis of another genetic disease called alpha-1 Antitrypsin deficiency (Kidd et al, Nature 304, 230, 1983). The oligonucleotide probes or their subsequent modifications can also be used for identification of mutant phenylalanine hydroxylase genes even if the molecular lesion does involve a restriction recognition sequence. If there are multiple mutations in the phenylalanine hydroxylase genes that can result in disease, the various mutant phenylalanine hydroxylase genes can be isolated from a number of affected individuals with different ethnic backgrounds and analyzed in the same manner. The identification of mutant sequences would lead to the synthesis of a battery of oligonucleotides or their derivatives that would permit the analysis of mutant phenylalanine hydroxylase genes in the population. This approach is particularly important in light of the fact that the detection of mutant phenylalanine hydroxylase genes in the general population can be performed in random individuals without history of classical PKU. Thus, this method of analysis can potentially be developed into a mass screening program for PKU carriers in the general population.

Use of Polymorphism in the Phenylalanine Hydroxylase Locus
for Linkage Analysis of Neighboring Genes Involved in
Other Diseases

The high level of polymorphisms in the human phenylalanine hydroxylase locus as dislcosed in this application is a very useful marker in the human genome. Using the human phenylalanine hydroxylase cDNA clone as a hybridization probe to analyze a panel of human/rodent hybrid cells containing an assortment of human chromosomes, the phenylalanine hydroxylase gene has been assigned to human chromosome 12 (Lidsky et al, American Journal of Human Genetics, Volume 36, 1984, in press). The phenylalanine hydroxylase DNA clones can be used as hybridization probes to analyze pedigrees of other genetic diseases with or without known causes. If the segregation of the phenlyalanine hydroxylase gene is concordant with any other disease state, the PAH DNA probes can be used for analysis of those diseases as in PKU. The phenylalanine hydroxylase DNA clones can also be used as a starting point for chromosome walking into the respective disease locus. A series of DNA probes neighboring the phenylalanine hydroxylase gene on chromosome 12 will become available for polymorphism analysis of the respective diseases during the chromosome walking protocol. This process will eventually lead to the disease loci themselves on chromosome 12, which could then be used as the best probes for analysis of the particular diseases by the same approach outlined for classical PKU in this application.

Various Methods of Detecting the Phenylalanine Hydroxylase
Gene in the Human Genome

The DNA sequences in the human phenylalanine hydroxylase cDNA clone, as well as the corresponding genomic DNA clones, and their modifications thereof, can be used as hybridization probes to detect the corresponding gene for analysis of classical PKU after labeling by radioactive means. In addition, chemically modified PAH DNA clones can be used as hybridization probes which can be detected subsequently by non-radioactive means such as fluorescent or colorimetric measurements (Langer-Safer, et al, Proc. Natl. Acad. Sci. U.S.A., 79, 4381, 1982; Leary, et al, Proc. Natl. Acad. Sci. U.S.A., 80, 4045, 1983). The same approach for analysis of PKU using phenylalanine hydroxylase DNA probes can also be applied directly to the analysis of the milder form of the disease known as hyperphenyalaninemia.

Use of Human Phenylalanine Hydroxylase cDNA Clones to Determine the Chromosomal Location of the PKU Locus

Digestion of human genomic DNA with Bam HI followed by Southern hybridization analysis revealed an intense hybridization band at 20.5 kb. Analysis of total mouse DNA under the same conditions yields an intense band at 10.4 kb and a weaker signal at 5.7 kb, suggesting a rather high level of sequence homology between the phenylalanine hydroxylase genes in the two species. Since the phenylalanine hydroxylase gene is a unique DNA sequence in the human genome with no apparent pseudogenes, the presence of the human phenylalanine hydroxylase gene in human/mouse hybrid cells can be detected because of the distinct fragment sizes of the human and mouse genes.

A clonal panel of 12 human/mouse hybrid cells was used to localize the phenylalanine hydroxylase gene on a particular human chromosome. High molecular weight DNA was isolated from each hybrid cell line, digested with Bam HI, and subjected to Southern hybridization analysis using $^{32}$P-labeled phPH72 DNA. Every hybrid cell contained the 10.4-kb and 5.7-kb mouse DNA bands, and the presence of the 20.5-kb human DNA fragments was found in some of the hybrid lines. A comparison of these data with the human chromosome contents in each of the hybrid lines as determined by karyotype and isozyme data has demonstrated complete segregation concordance between the phenylalanine hydroxylase gene and the presence of chromosome 12. The only other human chromosomes with relatively high segregation concordance with the phenylalanine hydroxylase gene were 8 (88%), 10 (92%), and 20 (85%).

To further substantiate the assignment of the phenylalanine hydroxylase gene to chromosome 12, an additional panel of five hybrids was analyzed. DNA samples from two hybrid lines (AHA 3d2-3 and AHA 16e-3) containing chromosome 12 showed the presence of the 20.5-kb human DNA fragment after digestion with Bam HI and Southern blot analysis. Complete segregation concordance is again observed only with chromosome 12, and the potential association with the other human chromosomes can be ruled out on the basis of their reduced levels of segregation concordance with the phenylalanine hydroxylase gene. These results confirmed the previous assignment of the phenylalanine hydroxylase gene to chromosome 12.

The hybrids 41pT2A, BDA 17b17, and BDA 17b17-1 contain the cellular oncogene Ki-ras DNA sequence that has been assigned to chromosome 12 and are each positive for the TPI-1 isozyme marker that is located at 12p13 (data not shown). However, they are negative for the PEP-B marked that is located at 12q21, indicating the presence of only a part of chromosome 12 in these cells as a result of translocation. All three hybrids gave no hybridization signal corresponding to the human phenylalanine hydroxylase gene. [These results suggest that the phenylalanine hydroxylase gene is located on the long arm of chromosome 12.] This possibility should be tested directly by subchromosomal localization of the phenylalanine hydroxylase gene by in situ hybridization.

Accordingly, the present invention is well suited to attain the objects and ends and has the advantages and features mentioned as well as others inherent therein.

While presently preferred embodiments of the invention have been given for purposes of disclosure, changes and modifications can be made which are within the spirit of the invention as defined by the scope of the appended claims.

-1-

## CLAIMS

1. A human full-length phenylalanine hydroxylase cDNA clone.

2. A labelled human cDNA full-length phenylalanine hydroxylase cDNA clone.

3. _E. Coli_ RRI (pHPAH 247) having the deposit accession number ATC 39644.

4. _E. Coli_ ED 8767 (chPAH 15) having the deposit accession number ATC 39660.

5. _E. Coli_ ED 8767 (chPAH 10) having the deposit accession number ATC 39659.

6. _E. Coli_ ED 8767 (chPAH 14) having the deposit accession number ATC 39661.

7. _E. Coli_ ED 8767 (chPAH 25) having the deposit accession number ATC 39658.

8. A recombinant plasmid containing a full-length human cDNA for phenylalanine hydroxylase inserted into a DNA vector.

9. A method of diagnosing phenylalanine hydroxylase deficiency in a human comprising:
   gene mapping utilizing one of a labelled, full-length, human phenylalanine hydroxylase cDNA clone, a human chromosomal phenylalanine hydroxylase gene and fragments thereof as a hybridization probe.

10. Chemically synthesized specific oligonucleotides of the mutant sequences in the genomic PKU locus of persons affected with PKU.

11. Chemically synthesized specific oligonucleotides of the normal sequences of the genomic PKU locus.

12. A method of identifying PKU effected fetuses and PKU trait carriers without a proband comprising:
gene analysis utilizing synthetic oligonucleotides or their derivatives.

13. A method of synthesizing a full-length human phenylalanine hydroxylase clone from human liver tissue comprising:
screening a recombinant λgt11 human liver library with a cloned partial-length phenylalanine hydroxylase cDNA probe,
isolating recombinant phage plaques yielding resultant hybridization signals,
culturing the recombinant phages and isolating resulting human DNA,
digesting the resulting recombinant DNA and determining sizes of inserted fragments of human DNA, and
selecting a resulting clone having a full-length human phenylalanine hydroxylase insert.

14. A method for determining the chromosomal location of the PKU locus in humans comprising:
gene mapping utilizing a labelled human phenylalanine hydroxylase cDNA clone as a hybridization probe.

15. A method of detecting carriers of the phenylalanine hydroxylase deficiency trait comprising:
gene mapping utilizing a labelled, hybridization probe selected from the group consisting of a full-length human phenylalanine hydroxylase cDNA clone, human chromosomal phenylalanine hydroxylase gene and fragments thereof.

16. A method of diagnosing phenylketonuria prenatally comprising:

cleaving fetal DNA with enzymes,

cleaving both parents' DNA with said enzymes,

cleaving DNA from effected individuals with said enzymes,

cleaving DNA from available uneffected siblings with said enzymes, and

comparing the polymorphism of the cleaved DNAs utilizing a full-length phenylalanine hydroxylase cDNA clone.

17. An isolated human chromosomal phenylalanine hydroxylase gene.

18. Isolated human chromosomal phenylalanine hydroxylase gene fragements effective for use as a hybridization probe in diagnosing phenylalanine hydroxylase deficiency in a human being.

19. A polynucleotide molecule which contains a genetic sequence selected from the group consisting of:

a) a sequence containing the sequence for human PAH,

b) a sequence hybridizing therewith, and

c) a sequence degenerate with said sequence a or b.

20. The molecule of Claim 19 wherein said genetic sequence codes for human PAH.

21. The molecule of Claim 20 wherein said genetic sequence is a human chromosomal gene for PAH.

22. The molecule of Claim 19 wherein said genetic sequence hybridizes with a sequence coding for human PAH.

23. The molecule of Claim 22 which is a partial-length sequence of human PAH.

24. The molecule of Claim 22 which is a fragment of the human chromosomal gene for PAH.

25. The molecule of Claims 19, 20, 21, 22, 23 or 24 having a detectable label.

26. A cloning vehicle containing any one of the molecules of Claims 19, 20, 21, 22, 23 or 24 which is present in a cloning or expression vehicle.

27. An expression vehicle containing any one of the molecules of Claims 19, 20, 21, 22, 23 or 24.

28. A cDNA molecule which contains a genetic sequence selected from the group consisting of:
   a) a sequence containing the sequence for human PAH,
   b) a sequence hybridizing therewith, and
   c) a sequence degenerate with said sequence a or b.

29. The cDNA molecule of Claim 28 wherein said sequence codes for human PAH.

30. The cDNA molecule of Claim 28 wherein said sequence hybridizes with sequence coding for human PAH.

31. The cDNA molecule of Claim 30 which comprises a partial-length sequence of human PAH.

32. The molecule of any of Claims 28, 29, 30 or 31 having a detectable label.

a)   screening a recombinant λgtll human liver library with a cloned partial-length phenylalanine hydroxylase cDNA probe,

b)   isolating recombinant phage plaques yielding resultant hybridization signals,

c)   culturing the recombinant phages and isolating resulting human DNA,

d)   digesting the resulting recombinant DNA and determining sizes of inserted fragments of human DNA, and

e)   selecting a resulting clone having a full-length human phenylalanine hydroxylase cDNA insert.

39.   A method of diagnosing human phenylalanine hydroxylase deficiency prenatally which comprises:

detecting a RFLP in the human phenylalanine hydroxylase locus using as hybridization probe the polynucleotide of any of Claims 19, 28 or 35.

40.   A method of detecting carriers of the phenylalanine hydroxylase deficiency trait which comprises detecting a RFLP in the human PAH locus using as a hybridization probe the polynucleotide of any of Claims 19, 28 or 35.

41.   A method of prenatally diagnosing and detecting carriers of a genetic disease having a genetically linked locus to human PAH which comprises:

detecting a RFLP in the human PAH locus using as a hybridization probe the polynucleotide of any of Claims 19, 28 or 35.

42.   A specific oligonucleotide comprising a mutant sequence in the genomic PKU locus.

43.   A specific oligonucleotide comprising a normal sequence in the genomic PAH locus.

44. A method of identifying PKU effected fetuses an
PKU trait carriers without a proband comprising:
utilizing the specific oligonucleotide of Claims 23
or 25 or derivatives thereof for gene analysis.

0161788

1/3

Fig. 1

Fig. 2

*Fig. 3*

A — Msp I — lanes 1 2 3 4 5
(I)23
(II & III)19
(II)4
(III)2.2
1.8
Kb

B
(II)
a
4   19
23
(I)
1.8 2.2   19
a b (III)

*Fig. 4*

A — Pvu II — lanes 1 2 3 4 5
(AI)19
(BI)11.5
(BII)9.1
(AII)6.0
Kb

B
a          b
19
6
9.1
11.5

THE HUMAN CHROMOSOMAL PHENYLALANINE HYDROXYLASE GENE

*Fig. 5*